# EUROPÄISCHE PATENTSCHRIFT

(11) **EP 0 783 606 B1**
(45) Veröffentlichungstag und Bekanntmachung des Hinweises auf die Patenterteilung: **26.05.2004**
(21) Anmeldenummer: 96926962.0
(22) Anmeldetag: 16.08.1996
(51) Int. Cl.: D01F 13/02

(54) **VERFAHREN ZUR REINIGUNG EINER WÄSSRIGEN LÖSUNG EINES TERTIÄREN AMINOXIDS**
PROCESS FOR PURIFYING A TERTIARY AMINOXIDE AQUEOUS SOLUTION
PROCEDE DE PURIFICATION D'UNE SOLUTION AQUEUSE D'UN OXYDE D'AMINE TERTIAIRE

(30) Priorität: 18.08.1995 AT 140095
(43) Veröffentlichungstag der Anmeldung: 16.07.1997
(73) Patentinhaber: LENZING AKTIENGESELLSCHAFT, 4860 Lenzing (AT)
(72) Erfinder: MÜLLEDER, Eduard, A-4030 Linz (AT); MANGENG, Bruno, A-4863 Seewalchen (AT); SCHWENNINGER, Franz, 7563 Königsdorf 255 (AT); MÄNNER, Johann, A-4852 Weyregg (AT)
(74) Vertreter: Schwarz, Albin, Dr.
(86) Internationale Anmeldenummer: PCT/AT1996/000148
(87) Internationale Veröffentlichungsnummer: WO 1997/007268

(56) Entgegenhaltungen:
- EP-A- 0 427 701
- EP-A- 0 488 988
- WO-A-93/11287

## Beschreibung

Die vorliegende Erfindung betrifft ein Verfahren zur Reinigung einer wäßrigen Lösung eines tertiären Aminoxides, welche Lösung Verunreinigungen enthält, die teilweise in gelöster Form und teilweise in ungelöster, kolloidaler Form vorliegen. Die vorliegende Erfindung betrifft insbesondere ein Verfahren zur Reinigung von Fällbädern des Aminoxidverfahrens.

Seit einigen Jahrzehnten wird nach Verfahren zur Herstellung cellulosischer Formkörper gesucht, welche das heute in großem Maßstab angewendete Viskoseverfahren ersetzen sollen. Als eine nicht zuletzt wegen einer besseren Umweltverträglichkeit interessante Alternative hat sich dabei herauskristallisiert, Cellulose ohne Derivatisierung in einem organischen Lösungsmittel aufzulösen und aus dieser Lösung Formkörper, z.B. Fasern, Folien und anderen Formkörpern, zu extrudieren. Solcherart extrudierte Fasern erhielten von der BISFA (The International Bureau for the Standardization of man made fibers) den Gattungsnamen Lyocell. Unter einem organischen Lösungsmittel wird von der BISFA ein Gemisch aus einer organischen Chemikalie und Wasser verstanden.

Es hat sich herausgestellt, daß sich als organisches Lösungsmittel insbesondere ein Gemisch aus einem tertiären Aminoxid und Wasser sehr gut zur Herstellung von cellulosischen Formkörpern eignet. Als Aminoxid wird dabei in erster Linie N-Methylmorpholin-N-oxid (NMMO) verwendet. Andere Aminoxide sind z.B. in der EP-A - 0 553 070 beschrieben. Ein Verfahren zur Herstellung formbarer Celluloselösungen ist z.B. aus der EP-A - 0 356 419 bekannt. Die Herstellung cellulosischer Formkörper unter Anwendung tertiärer Aminoxide wird allgemein als Aminoxidverfahren bezeichnet.

In der EP-A - 0 356 419 ist ein Aminoxidverfahren zur Herstellung spinnbarer Celluloselösungen beschrieben, welches als Ausgangsmaterial u.a. eine Suspension von Cellulose in flüssigem, wäßrigem N-Methylmorpholin-N-oxid (NMMO) verwendet. Dieses Verfahren besteht darin, daß die Suspension in einem Dünnschichtbehandlungsapparat einstufig und kontinuierlich in eine formbare Lösung übergeführt wird. Die formbare Lösung wird schließlich in einem Formwerkzeug, z.B einer Spinndüse, zu Filamenten versponnen, die durch ein Fällbad geführt werden.

Die Cellulose wird aus der Lösung in einem wäßrigen Spinnbad ausgefällt. Dabei reichert sich das spinnbad an Aminoxid an. Der Gehalt an Aminoxid in den dabei anfallenden Prozeßwässern beträgt bis zu 30 Gew.-%. Für die Wirtschaftlichkeit des Aminoxid-Verfahrens ist es von entscheidender Bedeutung, daß das Aminoxid nahezu vollständig zurückgewonnen und wiederverwendet wird.

Gleichzeitig mit dem Aminoxid reichern sich im Spinnbad auch farblose bis stark gefärbte Abbauprodukte aus dem Aminoxid-Verfahren an, die einerseits die Qualität der hergestellten Formkörper beeinträchtigen können und andererseits ein sicherheitsrisiko darstellen können, da das Aminoxid unter gewissen Bedingungen zu stark exothermen Zersetzungsreaktionen neigt. Diese Substanzen müssen vor der Aufkonzentrierung aus der wäßrigen Lösung des Aminoxides entfernt werden.

Die Lösungen des Aminoxides enthalten zumeist anorganische Kationen und Anionen, wie z.B. Kationen von Natrium, Calcium und Magnesium, bzw. Chlorid, Sulfat, etc., die zum Teil aus dem im Verfahren zur Herstellung cellulosischer Formkröper eingesetzten Zellstoff und andererseits aus anderen im Prozeß eingesetzten und gebildeten Substanzen stammen. Diese Ionen reichern sich bei der Eindampfung und Aufkonzentrierung der Lösungen zu wieder im Verfahren einsetzbaren konzentrierten wäßrigen Lösungen des Aminoxides an und führen in weiterer Folge zu Niederschlägen und Verkrustungen in der Eindampfanlage.

Auch Metallionen und Metallteilchen, die ebenfalls aus dem eingesetzten Zellstoff, aber auch aus im Verfahren eingesetzten metallischen Apparateteile stammen können, reichern sich in der Lösung an. Es ist bekannt, daß eben diese metallischen Teilchen und Ionen in Lösungen der Cellulose in wäßrigen Aminoxiden zu oftmals heftigen Abbaureaktionen sowohl des Aminoxides als auch der Cellulose führen können.

Farbstoffe, die z.B. aus dem Abbau von im Aminoxidverfahren eingesetzten Verbindungen zur stabilisierung der Celluloselösung, aus Begleitsubstanzen des eingesetzten Zellstoffes sowie insbesondere aus Abbaureaktionen des eingesetzten Aminoxides stammen, beeinträchtigen ebenfalls die Durchführung des Aminoxidverfahrens und insbesondere die Qualität der gemäß dem Verfahren hergestellten cellulosischen Formkörper.

In den Lösungen befinden sich weiters organische Anionen, die zum Beispiel aus Abbaureaktionen des tertiären Aminoxides und der Cellulose stammen.

Die aufgezählten Stoffe befinden sich zum Teil gelöst, zum Teil jedoch auch in kolloidaler Form, als sogenannte Trübstoffe, in der Lösung.

Um die Wirtschaftlichkeit und die Sicherheit des Aminoxidverfahrens zu gewährleisten, ist es nun notwendig, alle beschriebenen Störstoffe möglichst vollständig aus der zu reinigenden Lösung zu entfernen.

Zur Reinigung von wäßrigen Lösungen von tertiären Aminoxiden sind in der Literatur bereits zahlreiche Vorschläge gemacht worden:

Die DD-A-254 199 beschreibt ein Verfahren zur Reinigung wäßriger Lösungen von NNMO, gemäß welchem die Lösung Anionenaustauscher passiert, wobei in einer ersten Stufe der Anionenaustauscher ein mit tertiären Aminogruppen vom Typ -CH₂N(CH₃)₂ besetztes Austauscherharz eines Styrol/Divinylbenzol-Copolymerisates enthält und in einer zweiten Stufe als funktionelle Gruppe quaternäre Ammoniumgruppen vom Typ -CH₂N(CH₃)₃OH enthält. Es wird beschrieben, daß die zu reinigende NNMO-Lösung zu Beginn der Reinigung dunkel, nach der ersten Stufe braun bis gelb und nach der zweiten Stufe hellgelb bis wasserklar ist.

Durch den Einsatz eines Anionenaustauschers werden vorwiegend die in der Lösung vorhandenen Farbstoffe sowie organische und anorganische Anionen aus der Lösung entfernt.

In der EP-A-0 427 701 ist ein Verfahren zur Reinigung von wäßrigen Aminoxidlösungen beschrieben, gemäß welchem die Reinigung in einem einstufigen Verfahren mit einem Anionenaustauscher durchgeführt wird, der als funktionelle Gruppe ausschließlich quaternäre Tetralkylammoniumgruppen der Formel -CH₂-N⁺(CH ₃)₃X⁻ oder -CH₂-N⁺[(CH₃)₂(CH₂OH)]X⁻ aufweist, worin X⁻ das Anion einer anorganischen oder organischen Säure darstellt, worauf der Anionenaustauscher mit einer wäßrigen sauren Lösung regeniert wird. Das Anion X⁻ stammt vorzugsweise von einer flüchtigen Säure, insbesondere Kohlensäure, Ameisensäure oder Essigsäure. Diese Säuren werden auch zur Regenerierung des Anionenaustauschers vorgeschlagen.

Ein Nachteil der ausschließlichen Verwendung von Anionenaustauschern in der Reinigung von wäßrigen Aminoxid-Lösungen besteht darin, daß die solcherart behandelten Lösungen einen hohen pH-Wert aufweisen, der in weiterer Folge zu einem erhöhten Reinigungsaufwand führt. Dazu kommt, daß bei diesen vorbekannten Verfahren Alkali- und Erdalkalikationen sowie teilweise basische Abbauprodukte (Morpholin, N-methylmorpholin und andere Verbindungen) nicht aus der Lösung entfernt werden.

Die DD-A-274 435 beschreibt ein Verfahren zur Rückgewinnung von N-Methylmorpholin-N-oxid, indem man die wäßrigen NNMO-Lösungen über Austauschersäulen, die mit SO₃H-Gruppen enthaltendem Styrol/Divinylbenzol-Copolymerisat gefüllt sind, bis zur maximalen äquimolaren Beladung leitet und anschließend das NNMO durch äquimolare Mengen Natriumhydroxid verdrängt.

Die alleinige Verwendung eines Kationenaustauschers ist jedoch nicht zweckmäßig, da zahlreiche Störstoffe vom Kationenaustauscher nicht aus der Lösung entfernt werden können.

Weitere Methoden zur Behandlung von Aminoxid-hältigen Abwässern des Aminoxidverfahrens sind in der EP-A-0 468 951 beschrieben.

Im Gegensatz zu den bisher beschriebenen Vorschlägen, die auf der Behandlung der zu reinigenden Lösung mit mindestens einem Ionenaustauscher oder einem System mit zum Ionenaustausch befähigten funktionellen Gruppen beruhen, wird in der EP-A-0 488 988 ein Verfahren zur Reinigung einer wäßrigen NNMO-Lösung, insbesondere einer Spinnbadlösung, vorgestellt, bei welchem die Lösung mit Absorptionsmitteln in Kontakt gebracht und anschließend einer Filtration unterworfen wird. Als Adsorptionsmittel werden gemäß dieser Literatur vorzugsweise Aluminiumoxid, siliciumdioxid und/oder Kohle eingesetzt. Das am Adsorptionsmittel absorbierte NNMO wird nach der Adsorption mit Wasser ausgewaschen, während die zu entfernenden Störstoffe am Adsorptionsmittel verbleiben.

In der Internationalen Patentanmeldung WO 93/11287 wird vorgeschlagen, die Regenerierung eines zur Reinigung von wäßrigen Lösungen von tertiären Aminoxiden eingesetzten Anionenaustauschers mit einer wäßrigen Lösung einer starken anorganischen Säure und anschließend mit Natronlauge durchzuführen. Es wird ferner vorgeschlagen, die Lösung vor oder bevorzugt nach dem Passieren des Anionenaustauschers über einen Kationenaustauscher zu führen. Es wird beschrieben, daß bei Verwendung eines stark basischen Anionenaustauschers die durch das Überleiten der zu reinigenden Lösung entstehende Färbung des Austauscherharzes so stark sei, daß eine bloße Regenerierung mit Natronlauge nicht ausreichend ist, um das Harz wieder zu entfärben. Um die Kapazität des Harzes aufrecht zu erhalten, muß es daher zusätzlich mit einer starken anorganischen Säure behandelt werden.

In der WO 93/11287 wird weiters vorgeschlagen, in der Lösung suspendierte, feste Stoffe vor dem Passieren des Ionenaustauschers aus der Lösung zu entfernen. Dies kann mittels einer Filtration geschehen, wobei gemäß der WO 93/11287 eine grobe Filtration ausreichend ist. Eine Entfernung der im Fällbad enthaltenen kolloidalen Stoffe ist im Verfahren der WO 93/11287 nicht vorgesehen.

Versuche der Erfinder der vorliegenden Erfindung haben gezeigt, daß sämtliche Reinigungsverfahren, welche einen Ionenaustauscher verwenden, für eine großtechnische Aufarbeitung von Fällbädern nicht gut geeignet sind, da die Standzeiten des (der) Ionenaustauscher unbefriedigend kurz sind. Als Standzeit wird dabei jene Zeitspanne verstanden, nach welcher der Ionenaustauscher regeneriert werden muß.

Die Erfindung stellt sich die Aufgabe, ein Verfahren zur Reinigung von Fällbädern bereitzustellen, das die obigen Nachteile nicht aufweist und bei welchem die Standzeiten des (der) verwendeten Ionenaustauscher(s) verlängert sind.

Das erfindungsgemäße Verfahren zur Reinigung einer wäßrigen Lösung eines tertiären Aminoxides, welche Lösung Verunreinigungen enthält, die teilweise in gelöster Form und teilweise in ungelöster, kolloidaler Form vorliegen, ist gekennzeichnet durch die Kombination der Schritte, daß
(A) die in ungelöster, kolloidaler Form vorliegenden Verunreinigungen agglomeriert werden, indem der wäßrigen Lösung ein Flockungsmittel zugegeben wird, und durch Filtration im wesentlichen vollständig entfernt werden, und
(B) die im Schritt (A) erhaltene wäßrige Lösung mit einem Ionenaustauscher in Kontakt gebracht wird.

Es hat sich gezeigt, daß die Entfernung der ungelösten, in kolloidaler Form vorliegenden Verunreinigungen mittels der erfindungsgemäß vorgesehenen Agglomeration und Filtration, also der Trübstoffe, überraschenderweise die Standzeiten der Ionenaustauscher außerordentlich verlängert. Die Ionenaustauscher brauchen im erfindungsgemäßen Verfahren somit nicht so oft regeneriert werden, wie dies im Stand der Technik, d.h. ohne Abtrennung der kolloidalen Verunreinigungen, erforderlich ist.

Für die Filtration der agglomerierten Verunreinigungen hat sich eine Tiefenfiltration am besten bewährt.

In diesem Zusammenhang wird erwähnt, daß es ohne Agglomeration der kolloidalen Verunreinigungen nicht möglich ist, diese durch Filtration abzutrennen. Bei Anwendung einer Tiefenfiltration würden z.B. die nicht agglomerierten Teilchen ungehindert durch das Filtrationsmedium durchtreten und dadurch nachgeschaltete Ionenaustauscher verstopfen.

Die Tiefenfiltration ist besonders wirkungsvoll, wenn die Korngröße des bei der Tiefenfiltration eingesetzten Filtermediums in Flußrichtung kleiner wird, wobei als Filtermedium am besten Kies, Zellstoff, Sand, Bimsstein, Hydroanthrazit oder eine Kombination davon eingesetzt wird.

Die Agglomerierung wird vorgenommen, indem der wäßrigen Lösung ein Flockungsmittel zugegeben wird.

Flockungsmittel werden üblicherweise in ausschließlich wäßrigen Abtrennungssystemen verwendet, also z.B. bei der Abwasserbehandlung in der Mineralgewinnung. überraschend ist, daß Flockungsmittel auch in Fällbädern, welche ein tertiäres Aminoxid enthalten, wirken.

In der Papierindustrie eingesetzte polymere Flockungsmittel werden abhängig von der Molekülgröße oft auch als Retentionsmittel bezeichnet. Für die Zwecke dieser Anmeldung soll der Begriff "Flockungsmittel" auch Substanzen umfassen, die als Retentionsmittel bekannt sind.

Es hat sich gezeigt, daß Flockungsmittel die kolloidalen Stoffe eines gebrauchten Fällbades des Aminoxidverfahrens in ausgezeichneter Weise zu Teilchen agglomerieren können, die abfiltriert werden können.

Flockungsmittel, welche eine oligomere oder eine polymere Substanz enthalten, haben sich besonders gut bewährt.

Das Flockungsmittel enthält bevorzugt ein Polyacrylamid.

Weiters ist bevorzugt, daß das Flockungsmittel ein Kondensat aus Dicyandiamid und Formaldehyd enthält oder ein Polyaluminiumchlorid enthält.

Das Flockungsmittel wird am besten in einer Konzentration von 0,5 ppm bis 10 ppm, bezogen auf die gesamte Menge der wäßrigen Lösung, eingesetzt.

Eine bevorzugte Ausgestaltung des erfindungsgemäßen Verfahrens ist, dadurch gekennzeichnet, daß die im Schritt (A) erhaltene wäßrige Lösung vor dem Schritt (B) mit einem Adsorberharz in Kontakt gebracht wird.

Als Ionenaustauscher kann ein Anionenaustauscher und ein Kationenaustauscher vorgesehen sein.

Das erfindungsgemäße Verfahren eignet sich insbesondere zur Reinigung eines gebrauchten Fällbad, welches im Aminoxidverfahren anfällt und bis zu 30 Gew.-% Aminoxid enthält, wobei als Aminoxid am besten N-Methylmorpholin-N-oxid eingesetzt wird. Es hat sich überraschenderweise gezeigt, daß auch die Anwesenheit von 30 Gew.-% NMMO die Wirksamkeit des erfindungsgemäßen Verfahrens nicht beeinträchtigt.

Die Erfindung betrifft somit auch ein Verfahren zur Reinigung eines gebrauchten Fällbades, welches im Aminoxidverfahren anfällt, Verunreinigungen in gelöster und ungelöster, kolloidaler Form aufweist und bis zu 30 Gew.-% N-Methylmorpholin-N-oxid enthält, und ist gekennzeichnet durch die folgenden Schritte, daß
(A) dem Fällbad, gegebenenfalls nach Verdünnung, ein Flockungsmittel zugegeben wird, um die in ungelöster, kolloidaler Form vorliegenden Verunreinigungen zu agglomerieren;
(B) die agglomerierten Verunreinigungen mit einer Tiefenfiltration abgetrennt werden, wobei eine im wesentlichen klare Lösung erhalten wird;
(C) die im Schritt (B) erhaltene klare Lösung mit einem Adsorberharz in Kontakt gebracht wird, um gefärbte Stoffe zu entfernen, worauf
(D) die Lösung mit einem Anionenaustauscher und einem Kationenaustauscher in Kontakt gebracht wird, um die Lösung weiter zu reinigen.

Mit den nachfolgenden Beispielen wird die Erfindung noch näher beschrieben.

### Beispiel 1

### Messung der Trübung

Die Messung der Trübung der Lösungen erfolgte durch die Verhältnisbildung der Intensität des in die Lösung eintretenden Lichtstrahls und des austretenden Lichtstrahls. Eine darüberhinaus stattfindende Absorption von Licht durch z.B. gefärbte Substanzen, wurde durch die Messung von durch die Trübung verursachtem Streulicht kompensiert. Die erhaltenen Meßwerte wurden mit einer standardisierten Eichlösung abgeglichen und in FTU (Formazin Turbidity Units) angegeben.

Die folgenden Messungen wurden mit dem Trübungsmeßgerät TRM der Fa. Drott, Deutschland, durchgeführt:

Einem gebrauchten Fällbad des Aminoxidverfahrens, welches ca. 20 Gew.-% NMMO enthielt und eine Trübung von 5 FTU aufwies, wurde das Flockungsmittel Praestol BC 853 (kationisches Flockungsmittel aus kationisiertem Polyacrylamid; Hersteller: Fa. Stockhausen) bis zu einer Konzentration von 10 ppm zugegeben. Anschließend wurde die Lösung über ein Tiefenfilter mit einer Schüttung aus Bims (Korngröße 3-5 mm) und Quarzsand (Korngröße ca. 1 mm) filtriert. Die Lösung wies nach der Tiefenfiltration eine Trübung von lediglich 0,4 FTU auf.

Die filtrierte Lösung wurde anschließend über ein mit tertiären Aminogruppen modifiziertes Adsorberharz (Type XUS 40285.00, Hersteller: Fa. Dowex) geleitet. Das erhaltene Eluat wurde über einen quaternäre Ammoniumgruppen aufweisenden, stark basischen Anionenaustauscher (Typ Lewatit MO 500, Hersteller: Fa. Bayer) sowie über einen stark sauren Kationenaustauscher mit Sulfonsäuregruppen als funktionelle Gruppen (Typ Lewatit SM, Hersteller: Fa. Bayer) geführt.

Die Standzeit des Anionenaustauschers betrug bis zur notwendig gewordenen Regenerierung etwa 70 Stunden, die des Kationenaustauschers etwa 240 Stunden.

Zum Vergleich wurde dem Fällbad bei sonst identischer obiger Versuchs führung kein Flockungsmittel zugegeben. Die Trübung der Lösung nach der Tiefenfiltration war in diesem Fall 3 FTU.

Die Standzeit des Anionenaustauschers reduzierte sich bereits während des Reinigungszyklus drastisch auf etwa 45 Stunden, die des Kationenaustauschers auf rund 170 Stunden.

### Beispiel 2

Beispiel 1 wurde wiederholt, wobei jedoch die Flockungsmittel Melflock 113 S (ein Kondensationsprodukt aus Dicyandiamid und Formaldehyd, Hersteller: Fa. Trostberg), Alzofix P9 (ein Kondensationsprodukt aus Polyaluminiumchlorid und Dicyandiamid, Hersteller: Fa. Trostberg) und Kombinationen des im Beispiel 1 eingesetzten Praestol BC 853 mit Melflock 113 S und mit Alzofix P9 eingesetzt wurden. Die Ergebnisse waren ähnlich denen, die im Beispiel 1 angegeben sind.

## Patentansprüche

1. Verfahren zur Reinigung einer wäßrigen Lösung eines tertiären Aminoxides, welche Lösung Verunreinigungen enthält, die teilweise in gelöster Form und teilweise in ungelöster, kolloidaler Form vorliegen, **gekennzeichnet durch** die Kombination der Schritte, daß
(A) die in ungelöster, kolloidaler Form vorliegenden Verunreinigungen agglomeriert werden, indem der wäßrigen Lösung ein Flockungsmittel zugegeben wird, und **durch** Filtration im wesentlichen vollständig entfernt werden, und
(B) die im Schritt (A) erhaltene wäßrige Lösung mit einem Ionenaustauscher in Kontakt gebracht wird.

2. Verfahren nach Anspruch 1, **dadurch gekennzeichnet, daß** die agglomerierten Verunreinigungen mit einer Tiefenfiltration abfiltriert werden.

3. Verfahren nach Anspruch 1 oder 2, **dadurch gekennzeichnet, daß** das Flockungsmittel eine oligomere oder eine polymere substanz enthält.

4. Verfahren nach Anspruch 3, **dadurch gekennzeichnet, daß** das Flockungsmittel ein Polyacrylamid enthält.

5. Verfahren nach Anspruch 3, **dadurch gekennzeichnet, daß** das Flockungsmittel ein Kondensat aus Dicyandiamid und Formaldehyd enthält.

6. Verfahren nach Anspruch 3, **dadurch gekennzeichnet, daß** das Flockungsmittel ein Polyaluminiumchlorid enthält.

7. Verfahren nach Anspruch 1 oder 2, **dadurch gekennzeichnet, daß** das Flockungsmittel in einer Konzentration von 0,5 ppm bis 10 ppm, bezogen auf die gesamte Menge der wäßrigen Lösung, eingesetzt wird.

8. Verfahren nach Anspruch 1, **dadurch gekennzeichnet, daß** die im Schritt (A) erhaltene wäßrige Lösung vor dem Schritt (B) mit einem Adsorberharz in Kontakt gebracht wird.

9. Verfahren nach einem der Ansprüche 1 oder 8, **dadurch gekennzeichnet, daß** als Ionenaustauscher ein Anionenaustauscher und ein Kationenaustauscher vorgesehen ist.

10. Verfahren nach einem der Ansprüche 1 bis 9, **dadurch gekennzeichnet, daß** als wäßrige Lösung eines tertiären Aminoxides ein gebrauchtes Fällbad eingesetzt wird, welches im Aminoxidverfahren anfällt und bis zu 30 Gew.-% Aminoxid enthält.

11. Verfahren nach einem der Ansprüche 1 bis 10, **dadurch gekennzeichnet, daß** das tertiäre Aminoxid N-Methylmorpholin-N-oxid ist.

12. Verfahren zur Reinigung eines gebrauchten Fällbades, welches im Aminoxidverfahren anfällt, Verunreinigungen in gelöster und ungelöster, kolloidaler Form aufweist und bis zu 30 Gew.-% N-Methylmorpholin-N-oxid enthält, **gekennzeichnet durch** die folgenden Schritte, daß
(A) dem Fällbad, gegebenenfalls nach Verdünnung, ein Flockungsmittel zugegeben wird, um die in ungelöster, kolloidaler Form vorliegenden Verunreinigungen zu agglomerieren;
(B) die agglomerierten Verunreinigungen mit einer Tiefenfiltration abgetrennt werden, wobei eine im wesentlichen klare Lösung erhalten wird;
(C) die im Schritt (B) erhaltene klare Lösung mit einem Adsorberharz in Kontakt gebracht wird, um gefärbte Stoffe zu entfernen, worauf
(D) die Lösung mit einem Anionenaustauscher und einem Kationenaustauscher in Kontakt gebracht wird, um die Lösung weiter zu reinigen.

## Claims

1. A process for the purification of an aqueous solution of a tertiary amine oxide containing impurities partially present in a dissolved and partially in a non-dissolved, colloidal state, **characterized by** a combination of the steps of
(A) agglomerating the impurities present in a non-dissolved, colloidal state by adding a flocculant to the aqueous solution and removing them essentially completely by filtration, and
(B) contacting the aqueous solution obtained in step (A) with an ion exchanger.

2. A process according to claim 1, **characterized in that** the agglomerated impurities are filtered out by means of deep-bed filtration.

3. A process according to claim 1 or 2, **characterized in that** the flocculant contains an oligomer or a polymer substance.

4. A process according to claim 3, **characterized in that** said flocculant contains a polyacrylamide.

5. A process according to claim 3, **characterized in that** said flocculant contains a condensate of dicyandiamide and formaldehyde.

6. A process according to claim 3, **characterized in that** said flocculant contains a polyaluminiumchloride.

7. A process according to claim 1 or 2, **characterized in that** said flocculant is used in a concentration of from 0.5 ppm to 10 ppm, based on the total amount of the aqueous solution.

8. A process according to claim 1, **characterized in that** the aqueous solution obtained in step (A) is contacted with an adsorber resin before step (B).

9. A process according to any of claims 1 or 8, **characterized in that** as an ion exchanger an anion exchanger and a cation exchanger is provided.

10. A process according to any of claims 1 to 9, **characterized in that** as said aqueous solution of a tertiary amine oxide a spent precipitation bath produced in the amine-oxide process and containing up to 30% by weight of amine oxide is used.

11. A process according to any of claims 1 to 10, **characterized in that** said tertiary amine oxide is N-methylmorpholine-N-oxide.

12. A process for the purification of a spent precipitation bath produced in the amine-oxide process, comprising impurities in a dissolved and in a non-dissolved, colloidal state and containing up to 30% by weight of N-methylmorpholine-N-oxide, **characterized by** the following steps of
(A) adding a flocculant to said precipitation bath, optionally after diluting it, to agglomerate said impurities present in a non-dissolved, colloidal state;
(B) separating said agglomerated impurities by means of deep-bed filtration, a substantially clear solution being obtained;
(C) contacting said clear solution obtained in step (B) with an adsorber resin to remove dyed substances, and thereafter
(D) contacting said solution with an anion exchanger and a cation exchanger to further purify said solution.

## Revendications

1. Procédé de purification d'une solution aqueuse d'un oxyde d'amine tertiaire, contenant en solution des impuretés se présentant en partie sous forme dissoute, et en partie non-dissoute, colloidales, **caractérisé par** la combinaison des étapes selon lesquelles :
(A) les impuretés se présentant sous forme non-dissoute colloidale sont agglomérées, par le fait qu'on ajoute à la solution aqueuse un agent floculent et qu'on l'élimine pratiquement complètement par filtration, et
(B) la solution aqueuse obtenue à l'étape (A) est mise en contact avec un échangeur d'ions.

2. Procédé selon la revendication 1, **caractérisé en ce que** les impuretés agglomérées sont filtrées avec une filtration profonde.

3. Procédé selon la revendication 1 ou 2, **caractérisé en ce que** l'agent floculant contient une substance oligomère ou un polymère.

4. Procédé selon la revendication 19, **caractérisé en ce que** l'agent floculant contient une polyacrylamide.

5. Procédé selon la revendication 3, **caractérisé en ce que** l'agent floculant contient un condensat, formé de dicyandiamide et formaldéhyde.

6. Procédé selon la revendication 3, **caractérisé en ce que** l'agent floculant contient un polychlorure d'aluminium.

7. Procédé selon la revendication 1 ou 2, **caractérisé en ce que** l'agent floculant est utilisé en une concentration de 0,5 ppm à 10 ppm, se rapportant à la quantité totale de solution aqueuse.

8. Procédé selon la revendication 1, **caractérisé en ce que** la solution aqueuse, obtenue à l'étape (A), est mise en contact avec une résine absorbante, avant l'étape (B).

9. Procédé selon l'une des revendications 1 ou 8, **caractérisé en ce qu'**on prévoit comme échangeur d'ions un échangeur d'anions et un échangeur de cations.

10. Procédé selon l'une des revendications 1 à 9, **caractérisé en ce qu'**on utilise comme solution aqueuse d'un oxyde d'amine tertiaire, un bain de précipitation usagé, qui précipite dans un procédé à l'oxyde d'amine et qui contient jusqu'à 30% en poids d'oxyde d'amine.

11. Procédé selon l'une des revendications 1 à 10, **caractérisé en ce que** l'oxyde d'amine tertiaire est du N-méthylmorpholin-N-oxyde.

12. Procédé d'épuration d'un bain de précipitation usagé, qui se présente dans un procédé d'oxyde d'amine, présentant des impuretés se présentant sous forme dissoute et non-dissoute, colloïdales, et contenant de jusqu'à 30% en poids de N-méthylmorpholin-N-oxyde, **caractérisé par** les étapes de procédé suivantes, selon lesquelles :
(A) on ajoute au bain de précipitation, le cas échéant après dilution, un agent floculant pour agglomérer les impuretés se présentant sous la forme non dissoute colloïdales.
(B) les impuretés agglomérées sont séparées avec une filtration profonde, sachant qu'on obtient une solution pratiquement claire
(C) la solution claire, obtenue à l'étape (B), est mise en contact avec une résine absorbante, pour éliminer les substances colorées, à la suite de quoi
(D) la solution est mise en contact avec un échangeur d'ions et un échangeur de cations pour procéder à une épuration supplémentaire de cette solution.
